(19) Europäisches Patentamt
European Patent Office
Office européen des brevets

(11) Veröffentlichungsnummer: **0 086 273**
**B1**

(12) # EUROPÄISCHE PATENTSCHRIFT

(45) Veröffentlichungstag der Patentschrift: **10.04.85**

(51) Int. Cl.⁴: **C 07 C 148/00**, C 07 C 149/40

(21) Anmeldenummer: **82111885.8**

(22) Anmeldetag: **21.12.82**

(54) **Verfahren zur Herstellung von 2-Naphthyl-thioglykolsäure.**

(30) Priorität: **16.01.82 DE 3201187**

(43) Veröffentlichungstag der Anmeldung: **24.08.83 Patentblatt 83/34**

(45) Bekanntmachung des Hinweises auf die Patenterteilung: **10.04.85 Patentblatt 85/15**

(84) Benannte Vertragsstaaten: **CH DE FR GB IT LI**

(56) Entgegenhaltungen:
**DE - A - 1 804 266**
**US - A - 2 884 449**

**JOURNAL OF THE AMERICAN CHEMICAL SOCIETY, Band 82, 1960, Easton F.M. Furman et "Novel reactions of 2-naphthol with thioglycolic acid and other mercaptans"**
**INDIAN JOURNAL OF TECHNOLOGY, Band 13, Oktober 1975, New Delhi D.W. RANGNEKAR et al. "Technical preparation of vat brown RRD"**
**Chemical Abstracts, Band 85, Nr. 19, 8. November 1976, Columbus, Ohio, USA V. PONOMAREVA et al. "2-Napthylthioglycolic acid" Seite 507**

(73) Patentinhaber: **CASSELLA Aktiengesellschaft, Hanauer Landstrasse 526, D-6000 Frankfurt am Main 61 (DE)**

(72) Erfinder: **Bauer, Wolfgang, Dr., Masurenstrasse 6, D-6457 Maintal 3 (DE)**

(74) Vertreter: **Urbach, Hans-Georg, Dr., Hanauer Landstrasse 526, D-6000 Frankfurt am Main 61 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 086 273 B1

ACTORUM AG

## Beschreibung

Die Erfindung betrifft ein wirtschaftlich und ökologisch günstiges Verfahren zur Herstellung von 2-Naphthylthioglykolsäure der Formel I

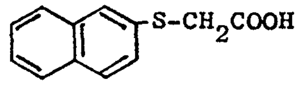

(I)

auch (2-Naphthylthio)essigsäure genannt, die z.B. als Zwischenprodukt für die Herstellung von Farbstoffen verwendet wird.

Zur Herstellung von 2-Naphthylthioglykolsäure sind verschiedene Verfahren bekannt – US-PS Nr. 2884449, „J. Amer. Chem. Soc.", *82*, 1450 (1960), „Indian Journal of Technology", *13*, 462, (1975), SU-PS Nr. 515743, „C. A.", *85*, 142911 b (1976) und „C.A.", *89*, 42865 d (1978) –, bei denen 2-Naphthol mit Thioglykolsäure (auch Mercaptoessigsäure genannt) in Gegenwart saurer Katalysatoren kondensiert wird. Die Verfahren unterscheiden sich in der Art der Durchführung der Kondensationsreaktion. Als saure Katalysatoren werden dabei z.B. Natriumhydrogensulfat, Alkansulfonsäure, p-Toluolsulfonsäure, Polyphosphorsäure, Schwefelsäure, HCl, Benzoesäure oder ein saurer Kationenaustauscher (SU-PS Nr. 515743) verwendet. Die Kondensation wird dabei ohne Lösungsmittel (US-PS Nr. 2884449, „J. Amer. Chem. Soc.", loc. cit., „Indian Journal of Technology", loc. cit.) oder in Benzol oder Chlorbenzol als Lösungsmittel („Indian Journal of Technology", loc. cit.) durchgeführt. Zum Teil werden wasserfreie Bedingungen eingehalten (SU-PS Nr. 2884449, „J. Amer. Chem. Soc.", loc. cit.), zum Teil wird in Gegenwart von Wasser kondensiert (US-PS Nr. 515743 und „C.A.", *89*, 42865d). Bei allen diesen Verfahren ist es nachteilig, dass die bei der Kondensation entstehende 2-Naphthylthioglykolsäure neben anderen Verunreinigungen noch erhebliche Mengen nicht umgesetztes 2-Naphthol enthält und dass zu dessen Abtrennung grosse Reaktionsvolumina erforderlich sind und grosse Abwassermengen anfallen, die zudem schlecht chemisch und biologisch abbaubar sind. Durch die für die Reinigung erforderlichen grossen Reaktionsvolumina ergibt sich eine schlechte Raumausbeute. Bei der Verwendung von Benzol oder Chlorbenzol als Lösungsmittel bei der Kondensation werden nur unbefriedigende Ausbeuten von 27,5 bzw. 31,1% der Theorie erhalten (vgl. „Indian Journal of Technology", loc. cit., S. 463, Tabelle 2).

Es wurde nun überraschenderweise gefunden, dass 2-Naphthylthioglykolsäure durch säurekatalysierte Kondensation von 2-Naphthol mit Thioglykolsäure durch säurekatalysierte Kondensation von 2-Naphthol mit Thioglykolsäure mit hoher Reinheit, hoher Raumausbeute und einer theoretischen Ausbeute von 95 bis 98% hergestellt werden kann, wenn das bei der Kondensation entstehende Kondensationswasser aus dem Reaktionsgemisch mit Hilfe eines Lösungsmittels entfernt wird, das mit Wasser ein Azeotrop bildet.

Die Entfernung des bei der Kondensation entstehenden Wassers kann dabei z.B. durch azeotrope Destillation oder durch Kochen am Rückfluss unter Einschaltung eines Wasserabscheiders erfolgen.

Bei dem erfindungsgemässen Verfahren wird die Kondensation von 2-Naphthol mit Mercaptoessigäure bei Temperaturen von 105 bis 160 °C, vorzugsweise 125 bis 145 °C, durchgeführt. Dabei betragen die Reaktionszeiten, je nach dem benutzten Katalysator, ca. 2 bis 14 h.

Geeignete Lösungsmittel, die mit Wasser azeotrope Gemische bilden, sind z.B. in Wasser nicht oder nur wenig lösliche organische Lösungsmittel, wie Kohlenwasserstoffe, wie z.B. Octan, Isooctan, Nonen, Nonan, Methylcyclohexan, Cumol, Toluol, o- und m-Xylol und Halogen/Kohlenwasserstoffe, wie z.B. 1.1.1.2-Tetrachlorethan, 1.1.2.2-Tetrachlorethan, 1.1.2-Trichlortrifluorethan, Chlorbenzol, o- und m-Dichlorbenzol, 2-, 3- und 4-Chlortoluol, Pentachlorethan, Tetrachlorethylen und z.B. Nitrobenzol. Das Lösungsmittel sollte gegenüber den Reaktionskomponenten inert sein und, damit die Reaktion unter Normaldruck durchgeführt werden kann, einen genügend hohen Siedepunkt besitzen. Es kann auch ein Gemisch verschiedener geeigneter Lösungsmittel verwendet werden. Falls das bei der Kondensation entstehende Wasser mit dem Lösungsmittel azeotrop abdestilliert wird, braucht nur so wenig Lösungsmittel zugefügt zu werden, wie für die azeotrope Abdestillation des Wassers erforderlich ist. Nachdem das bei der Kondensation zwischen 2-Naphthol und Thioglykolsäure gebildete Kondensationswasser entfernt worden ist, braucht kein Lösungsmittel mehr im Reaktionsgemisch anwesend zu sein. Normalerweise wird auch dann, wenn alles Lösungsmittel azeotrop abdestilliert worden ist, die Reaktionsmischung noch weiter erhitzt.

Falls das bei der Kondensation entstehende Kondensationswasser durch Rückflussdestillation unter Einschaltung eines Wasserabscheiders entfernt werden soll, ist die Verwendung eines Lösungsmittels erforderlich, aus dem sich im Wasserabscheider das Wasser in einer getrennten Schicht abtrennt.

Als Katalysatoren bei dem erfindungsgemässen Verfahren werden saure Katalysatoren, insbesondere starke, nicht oxidierend und sulfonierend wirkende Säuren verwendet, beispielsweise: Methansulfonsäure, Ethansulfonsäure, Propan-1-sulfonsäure, Butan-1-sulfonsäure, 2-Methylpropan-2-sulfonsäure, Pentan-1-sulfonsäure, Hexan-1-sulfonsäure, Gemische von Alkansulfonsäuren, Methandisulfonsäure, Perfluoressigsäure, Perfluoralkansulfonsäuren, beispielsweise Perfluormethansulfonsäure, Perfluorbutansulfonsäure, perfluorierte Polymere mit Sulfonsäuregruppen (Nafion H; Hersteller: Du Pont), sowie Arylsulfonsäuren, beispielsweise Benzolsulfonsäure, p-Toluolsulfonsäure, o-Xylolsulfonsäure-(4), m-Xylolsulfonsäure-(4), p-Xylolsulfonsäure-(2). Es kann auch ein Katalysatorgemisch verwendet werden. Von dem Katalysator werden normalerweise 0,01 bis 0,5 mol, vorzugsweise 0,02 bis 0,15 mol pro Mol 2-Naphthol eingesetzt. Das Molverhältnis

der eingesetzten Ausgangskomponenten 2-Naphthol/Thioglykolsäure beträgt normalerweise 1:(1 bis 1,2), vorzugsweise 1:(1,1 bis 1,15). Die Anwendung eines grösseren Überschusses an Thioglykolsäure bringt keine Vorteile.

Nachdem aus „Indian Journal of Technology", *13*, 462 (1975) bekannt war, dass die in Chlorbenzol bei 130 °C mit p-Toluolsulfonsäure als Katalysator durchgeführte Kondensation von 2-Naphthol mit Thioglykolsäure (Reaktionszeit: 16 h) lediglich eine Ausbeute von 31% der Theorie an 2-Naphthylthioglykolsäure liefert, war es ausserordentlich überraschend und auch für den Fachmann nicht vorhersehbar, dass die ebenfalls in Chlorbenzol bei 130 °C unter azeotroper Destillation durchgeführte Reaktion eine Ausbeute von 97% der Theorie an 2-Naphthylthioglykolsäure liefert. Nach dem erfindungsgemässen Verfahren wird 2-Naphthol praktisch quantitativ umgesetzt, so dass die nach den bisher bekannten Verfahren erforderliche Aufwendige, mit sehr schlechter Raumausbeute verbundene Abtrennung von 2-Naphthol entfallen kann. Nach dem erfindungsgemässen Verfahren wird 2-Naphthylthioglykolsäure mit einem hohen Reinheitsgrad erhalten, so dass keine weitere Reinigungsoperation durch Umkristallisation erforderlich ist. Weitere Vorteile des erfindungsgemässen Verfahrens bestehen darin, dass ökologisch wesentlich günstigere Fabrikationsabwässer und keine Fabrikationsrückstände anfallen, was im Hinblick auf den Umweltschutz von erheblicher Bedeutung ist.

Die folgenden Beispiele dienen zur weiteren Erläuterung des erfindungsgemässen Verfahrens. Die Gehaltsbestimmungen von 2-Naphthylthioglykolsäure wurden acidimetrisch unter potentiometrischer Anzeige durchgeführt. Die Gehaltsbestimmung von 2-Naphthol in den isolierten, getrockneten Reaktionsprodukten erfolgte durch quantitative Dünnschichtchromatographie mit einer Fehlergrenze von 0,5%. Die Ausbeuteangaben in Prozent der Theorie beziehen sich auf eingesetztes 2-Naphthol und unter Berücksichtigung des Reinheitsgrads auf 2-Naphthylthioglykolsäure 100%ig gerechnet. Die angegebenen Schmelzpunkte sind nicht korrigiert.

*Beispiel 1*

Eine Mischung von 144,2 g 2-Naphthol, 106,9 g Thioglykolsäure 99%ig und 17,2 g p-Toluolsulfonsäure wird in 50 ml Chlorbenzol im Verlauf von 1 h unter Stickstoffatmosphäre auf 130 bis 135 °C geheizt, wobei unter Rühren das Chlorbenzol/Wasser azeotrop abdestilliert wird. (Aus dem Destillat trennen sich ca. 18 ml Wasser ab.) Anschliessend lässt man ca. 14 h bei 130 bis 135 °C nachrühren, kühlt auf 100 °C und rührt die Reaktionsschmelze in 1,2 l Wasser ein. Man rührt die wässerige Suspension ca. 1 h bei 20 bis 30 °C nach, filtriert und wäscht den Filterkuchen mit 200 ml Wasser nach. Die erhaltene 2-Naphthylthioglykolsäure wird bei 70 °C und einem Druck von ca. 135 mbar getrocknet.

Ausbeute: 220,6 g helles Pulver
Schmelzpunkt: 90 bis 91 °C
Gehalt an 2-Naphthylthioglykolsäure: 96%
Gehalt an 2-Naphthol: <0,5%
Ausbeute, bezogen auf eingesetztes 2-Naphthol: 97% der Theorie
Volumen des Filtrats: ca. 1,5 l

*Beispiel 2*

144,2 g 2-Naphthol, 106 g Thioglykolsäure 99%ig und 13,7 g Methansulfonsäure 70%ig werden in 50 ml Chlorbenzol eingetragen und unter Stickstoffatmosphäre auf 135 bis 140 °C erhitzt, wobei Wasser azeotrop abdestilliert (man erhält ca. 22 ml Wasser). Anschliessend lässt man mehrere Stunden bei 135 °C nachreagieren. Die auf ca. 100 °C gekühlte Schmelze wird anschliessend zu einer Mischung von 110 ml 10N-Natronlauge und 2,5 l Wasser gegeben. Die wässerige Lösung des 2-Naphthylthioglykolsäure/Natriumsalzes wird filtriert (in dem geringen Filterrückstand ist chromatographisch kein 2-Naphthol nachzuweisen). Das Filtrat wird mit verdünnter Schwefelsäure bis zu einem pH-Wert von 2 angesäuert. Die ausgefallene 2-Naphthylthioglykolsäure wird bei ca. 20 °C durch Filtration isoliert, mit 250 ml Wasser gewaschen und bei 70 °C und einem Druck von ca. 135 mbar getrocknet.

Ausbeute: 209,5 g helles Pulver
Schmelzpunkt: 91 bis 92 °C
Gehalt an 2-Naphthylthioglykolsäure: 99%
Gehalt an 2-Naphthol: < 0,5%
Ausbeute, bezogen auf eingesetztes 2-Naphthol: 99% der Theorie
Volumen des Filtrats: ca. 3,3 l

Setzt man anstelle von Monochlorbenzol bei den Beispielen 1 und 2 andere mit Wasser ein Azeotrop bildende Lösungsmittel, beispielsweise Toluol, o-Xylol, m-Xylol, p-Xylol, o-, m- oder p-Chlortoluol, o-Dichlorbenzol, ein, erhält man 2-Naphthylthioglykolsäure ebenfalls in ausgezeichneter Ausbeute und Qualität. In der folgenden Tabelle sind weitere Beispiele angegeben, die entsprechend dem in Beispiel 1 beschriebenen Verfahren durchgeführt werden, wobei die in Spalte 2 aufgeführten, mit Wasser ein Azeotrop bildenden Lösungsmittel und die in Spalte 3 aufgeführten Katalysatoren eingesetzt werden. In der Tabelle sind weiterhin angegeben:

in Spalte 4: die Reaktionstemperatur (°C)
in Spalte 5: die Reaktionszeit in Stunden
in Spalte 6: die Ausbeute in Prozent der Theorie
in Spalte 7: der Schmelzpunkt (°C)
in Spalte 8: der Gehalt des isolierten Produkts an 2-Naphthol (%)

*(Tabelle auf der nächsten Seite)*

*Beispiel 9*

Eine Mischung von 144,2 g 2-Naphthol, 106 g Thioglykolsäure und 17,2 g p-Toluolsulfonsäure in 200 ml p-Xylol wird in einer mit Rückflusskühler und einem Wasserabscheider versehenen Reaktionsapparatur auf 136 bis 138 °C erhitzt. Im Verlauf von ca. 3 h scheiden sich im Wasserabscheider 18 ml Wasser ab. Man rührt noch 5 h bei 136 bis 138 °C nach und entfernt dann das Lösungsmittel

| Beispiel Nr. | Lösungsmittel (Menge) | Katalysator (Menge) | Reaktionstemperatur (°C) | Reaktionszeit (h) | Ausbeute (% der Theorie) | Schmelzpunkt (°C) | Gehalt an 2-Naphthol (%) |
|---|---|---|---|---|---|---|---|
| 3 | o-Xylol (60 ml) | Propan-1-sulfonsäure (0,1 mol) | 130-140 | 12 | 96 | 90-92 | < 0,5 |
| 4 | Chlorbenzol (70 ml) | Trifluoressigsäure (0,1 mol) | 135-140 | 14 | 95 | 90-91 | < 1 |
| 5 | 2-Chlortoluol (50 ml) | p-Toluolsulfonsäure (0,1 mol) | 135-140 | 15 | 97 | 90-92 | < 0,5 |
| 6 | Chlorbenzol (50 ml) | Methandisulfonsäure (0,05 mol) | 130-135 | 13 | 97 | 91-92 | < 0,5 |
| 7 | Toluol (50 ml) | Perfluorbutansulfonsäure (0,025 mol) | 130-135 | 2 | 96 | 90-91 | < 0,5 |
| 8 | Chlorbenzol (50 ml) | Trifluormethansulfonsäure (0,03 mol) | 130-135 | 3 | 97 | 90-91 | < 0,5 |

durch Destillation. Anschliessend kühlt man auf 100 °C und rührt die Reaktionsschmelze in 1,2 l Wasser ein. Die erhaltene Suspension wird bei 20 °C filtriert und mit 200 ml Wasser gewaschen. Das Produkt wird bei 70 °C und einem Druck von ca. 135 mbar getrocknet.

Ausbeute: 218 g helles Pulver
Schmelzpunkt: 89 bis 91 °C
Gehalt an 2-Naphthylthioglykolsäure: 96%
Gehalt an 2-Naphthol: <0,5%
Ausbeute, bezogen auf eingesetztes 2-Naphthol: 96% der Theorie
Volumen des Filtrats: ca. 1,5 l

In den folgenden Vergleichsbeispielen werden sauer katalysierte Reaktionen von 2-Naphthol mit Thioglykolsäure beschrieben, welche dem bisherigen Stand der Technik entsprechen und ohne ein mit Wasser ein Azeotrop bildendes Lösungsmittel durchgeführt werden.

*Vergleichsbeispiel 1*

144,2 g 2-Naphthol, 102 g Thioglykolsäure 99%ig und 100 g $NaHSO_4 \cdot H_2O$ werden unter Stickstoffatmosphäre vermischt und 14 h bei 115 °C gerührt (in der mit einer Destillationsbrücke versehenen Apparatur geht kein Reaktionswasser über). Man gibt 400 ml Wasser zu, kühlt auf Raumtemperatur und filtriert. Das Rohprodukt wird in einer Mischung von 9000 ml Wasser und 240 g 20%iger Natronlauge gelöst. Anschliessend wird durch Einleiten von $CO_2$ ein pH-Wert von 7,5 bis 8 eingestellt und bei 60 °C filtriert. Das Filtrat wird mit 10N-Salzsäure auf pH 2 gestellt. Das ausgefallene Produkt wird bei 20 °C durch Filtration isoliert, mit 200 ml Wasser gewaschen und bei 70 °C und einem Druck von ca. 135 mbar getrocknet.

Ausbeute: 141 g helles Pulver
Schmelzpunkt: 84 bis 87 °C
Gehalt an 2-Naphthylthioglykolsäure: 62%
Gehalt an 2-Naphthol: ca. 40%
Ausbeute, bezogen auf eingesetztes 2-Naphthol: 40% der Theorie
Volumen des Filtrats: ca. 9,2 l

Führt man die Reaktion bei einer Temperatur von 135 bis 140 °C und einer Reaktionszeit von 14 h durch, erhält man 2-Naphthylthioglykolsäure mit einem Reingehalt von 82%, einem 2-Naphtholgehalt von ca. 15% und in einer Ausbeute von 56% der Theorie, bezogen auf eingesetztes 2-Naphthol.

*Vergleichsbeispiel 2*

Eine Mischung von 144,2 g 2-Naphthol, 101,5 g Thioglykolsäure 99%ig und 25 g p-Toluolsulfonsäure wird unter Stickstoffatmosphäre 16 h bei 115 bis 120 °C gerührt. Dann wird die Schmelze mit 10 l Wasser verdünnt, bei 60 °C mit 20%iger Natronlauge auf pH 8 gestellt und filtriert. Das Filtrat wird mit 2M-Schwefelsäure auf pH 2 gestellt. Das Reaktionsprodukt wird bei 20 °C filtriert, mit 250 ml Wasser säurefrei gewaschen und bei 70 °C und einem Druck von ca. 135 mbar getrocknet.

Ausbeute: 192 g helles Pulver
Schmelzpunkt: 87 bis 89 °C
Gehalt an 2-Naphthylthioglykolsäure: 89%
Gehalt an 2-Naphthol: ca. 5%
Ausbeute, bezogen auf eingesetztes 2-Naphthol: 78,4% der Theorie
Volumen des Filtrats: ca. 10,5 l

*Vergleichsbeispiel 3*

Eine Mischung von 144,2 g 2-Naphthol, 102,5 g Thioglykolsäure 99%ig und 15 ml 96%ige Schwefelsäure wird 16 h bei 115 °C unter Stickstoff gerührt. Anschliessend werden 8,5 l Wasser und 10N-Natronlauge bis zu einem pH-Wert von 9 zugegeben. Darauf leitet man bei 60 °C Kohlendioxid bis zu einem pH-Wert von 7,5 ein. Man filtriert bei 60 °C, stellt mit 10N-Salzsäure einen pH-Wert von 2 ein und isoliert das ausgefallene Produkt durch Filtration bei 20 °C. Man wäscht mit 350 ml Wasser säurefrei und trocknet den Presskuchen bei 70 °C und einem Druck von ca. 135 mbar.

Ausbeute: 196 g helles Pulver
Schmelzpunkt: 84 bis 86 °C

Gehalt an 2-Naphthylthioglykolsäure: 90%
Gehalt an 2-Naphthol: ca. 5%
Ausbeute, bezogen auf eingesetztes 2-Naphthol: 81% der Theorie
Volumen des Filtrats: ca. 9 l
Das Filtrat ist ökologisch ungünstig; chromatographisch sind 2-Naphtholsulfonsäuren deutlich nachweisbar.

*Vergleichsbeispiel 4*

Ein Gemisch von 144,2 g 2-Naphthol, 142 g Thioglykolsäure 99%ig und 45 g Ionenaustauscherharz (stark saurer Ionenaustauscher; Austauschkapazität 4,5 mEq/g; Hersteller: Merck AG) wird mit 56 ml Wasser versetzt und im Stickstoffstrom 6 h bei 135 °C gerührt. Man versetzt die Mischung mit 7,5 l Wasser und stellt mit ca. 190 ml 10N-NaOH auf pH 8. Anschliessend wird filtriert und das schwach alkalische Filtrat mit 2M-Schwefelsäure auf pH 2 gestellt. Das Produkt wird bei 20 °C filtriert, säurefrei gewaschen und bei 70 °C und einem Druck von ca. 135 mbar getrocknet.
Ausbeute: 163,5 g
Schmelzpunkt: 79 bis 81 °C
Gehalt an 2-Naphthylthioglykolsäure: 90%
Gehalt an 2-Naphthol: ca. 10%
Ausbeute, bezogen auf eingesetztes 2-Naphthol: 67% der Theorie
Volumen des Filtrats: ca. 8 l

## Patentansprüche

1. Verfahren zur Herstellung von 2-Naphthylthioglykolsäure durch säurekatalysierte Kondensation von 2-Naphthol mit Thioglykolsäure bei erhöhter Temperatur, dadurch gekennzeichnet, dass das bei der Reaktion entstehende Kondensationswasser aus dem Reaktionsgemisch mit Hilfe eines Lösungsmittels entfernt wird, das mit Wasser ein Azeotrop bildet.

2. Verfahren nach Anspruch 1, dadurch gekennzeichnet, dass die Kondensation in einem Temperaturbereich von 105 bis 160 °C durchgeführt wird.

3. Verfahren nach den Ansprüchen 1 und/oder 2, dadurch gekennzeichnet, dass die Kondensation in einem Temperaturbereich von 125 bis 145 °C durchgeführt wird.

4. Verfahren nach einem oder mehreren der Ansprüche 1 bis 3, dadurch gekennzeichnet, dass man als Katalysatoren starke, nicht oxidierende und sulfonierende Säuren einsetzt.

5. Verfahren nach einem oder mehreren der Ansprüche 1 bis 4, dadurch gekennzeichnet, dass als Lösungsmittel, das mit Wasser ein Azeotrop bildet, ein in Wasser schwer oder unlösliches organisches Lösungsmittel verwendet wird.

## Revendications

1. Procédé de préparation de l'acide (naphtyl-2)-thioglycolique par condensation, catalysée au moyen d'un acide, du naphtol-2 avec l'acide thioglycolique, à température élevée, procédé caractérisé en ce que l'eau engendrée par la condensation est chassée du mélange réactionnel au moyen d'un solvant formant un azéotrope avec l'eau.

2. Procédé selon la revendication 1, caractérisé en ce qu'on effectue la condensation dans un intervalle de température allant de 105 à 160 °C.

3. Procédé selon les revendications 1 et/ou 2, caractérisé en ce qu'on effectue la condensation dans un intervalle de température allant de 125 à 140 °C.

4. Procédé selon l'une quelconque des revendications 1 à 3, caractérisé en ce qu'on utilise, comme catalyseurs, des acides forts non oxydants et capables de sulfoner.

5. Procédé selon l'une quelconque des revendications 1 à 4, caractérisé en ce qu'on utilise, comme solvant formant un azéotrope avec l'eau, un solvant organique peu soluble ou insoluble dans l'eau.

## Claims

1. Process for the preparation of 2-naphthylthioglycolic acid by acid-catalysed condensation of 2-naphthol with thioglycolic acid at an elevated temperature, characterised in that the water of condensation being produced in the reaction is removed from the reaction mixture with the aid of a solvent which forms an azeotrope with water.

2. Process according to Claim 1, characterised in that the condensation is carried out in a temperature range from 105 to 160 °C.

3. Process according to Claims 1 and/or 2, characterised in that the condensation is carried out in a temperature range from 125 to 145 °C.

4. Process according to one or more of Claims 1 to 3, characterised in that strong non-oxidising and sulphonating acids are employed as the catalysts.

5. Process according to one or more of Claims 1 to 4, characterised in that an organic solvent, which is sparingly soluble or insoluble in water, is used as the solvent forming an azeotrope with water.